# EUROPEAN PATENT APPLICATION

(11) **EP 1 972 284 A2**
(43) Date of publication of application: **24.09.2008**
(21) Application number: 08250449.9
(22) Date of filing: 07.02.2008
(51) Int. Cl.: A61B 17/02, G05G 5/06

(54) **Retractor ring holder**

(30) Priority: 07.02.2007 US 888668 P
(71) Applicant: Codman & Shurtleff, Inc., Raynham, Massachusetts 02767-0350 (US)
(72) Inventor: Agbodoe, Victor, Stoughton, MA 02072 (US); Storz, Olaf, 78532 Tuttlingen (DE); Doherty, Joseph, Hopkinton, MA 01748 (US); Bookwalter, John, Putney, VT 05346 (US)
(74) Representative: Tunstall, Christopher Stephen

(57) **Abstract**

A retractor ring holder includes a bar portion and a ring holder portion. A distal end of the ring holder portion having a fixed clamp portion and a moveable clamp portion. An annual mating saw connection is disposed between the distal end of the bar portion and the proximal end of the ring holder portion. A first handle is attached to the connection to selectively place the bar portion and the ring holder portion in one of a fixed position with respect to each other and a moveable position. In the moveable position the ring holder portion is free to rotate 360° with respect to the bar portion. A second handle is attached to the ring holder portion proximal end to selectively move the moveable clamp portion with respect to the fixed clamp portion.

## Description

### FIELD OF THE INVENTION

The present invention relates to methods and devices for holding a retractor ring.

### BACKGROUND OF THE INVENTION

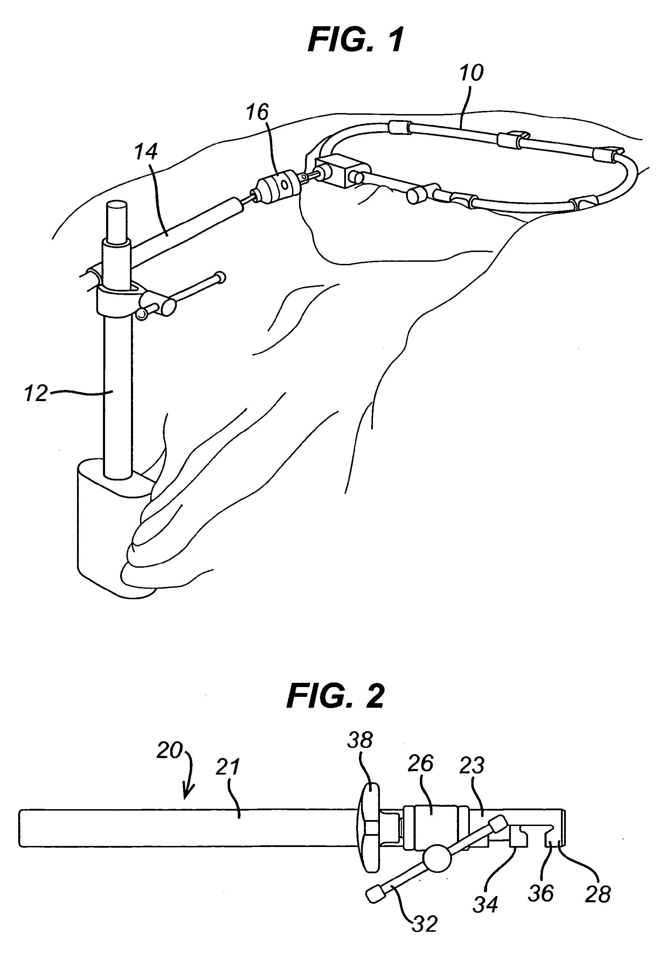

During surgery, retractors, such as those found in the BOOKWALTER^{™} retractor kit, which is commercially available from Codman & Shurtleff, Inc. of Raynham, MA, are often used to assist the surgeon and other operating room personnel to provide exposure to the surgical site for a broad range of surgical procedures. In surgical operations of the chest or abdomen, for example, it is often necessary to use a retraction apparatus to retain tissue away from the operative site. Typically, the retraction apparatus includes a housing member configured to lock onto a circumferential ring 10 located above the operative site (see Fig. 1). To maintain ring 10 in a fixed position, ring 10 can be connected to a support post 12 adjacent to the site by a horizontal bar 14 that has a flexible joint 16 (horizontal flex bar) to permit ring 10 to be oriented in various positions, as shown in Fig. 1. A horizontal extension arm may also be attached to the support post for supporting the circumferential ring. Within the housing member a retraction blade can usually be found for grabbing the tissue around the surgical incision. The housing member can also include a ratcheting mechanism and/or a tilting mechanism to draw the retraction blade away from the incision, thereby effecting the pulling away and/or lifting of the tissue around the incision to expose the desired surgical area. Examples of such retractor systems are disclosed in U.S. Pat. Nos. 4,254,763, 4,424,724 and 5,375,481, the disclosures of which are hereby incorporated by reference. During open surgical operations, such as, for example, open bariatric, ALIF procedures, hepatic resections, transplant procedures, abdominal aortic aneurysms, hernia repair, appendectomy and others, many different instruments are used, such as, for example, a retractor blade with ring attachment systems are used. Sometimes the weight of these instruments in combination with the force applied by the retractor blades is sometimes so great as to cause the ring to move with respect to the horizontal flex bar. Additionally, if the ring is unintentionally bumped, the ring may also move from its desired postion.

Thus, despite these retraction systems, there continues to be a need for a retraction system that can securely hold a retractor ring while permitting the ring to rotate 360 degrees. It would therefore be advantageous to have a retractor ring holder that provides a holding force up to about 250 pounds, while still permitting the ring to rotate 360 degrees.

### SUMMARY OF THE INVENTION

### BRIEF DESCRIPTION OF THE DRAWINGS

The invention will be more fully understood from the following detailed description taken in conjunction with the accompanying drawings, in which:

FIG. 1 is a plan view of a conventional ring holder;

FIG. 2 is a plan view of the ring holder in accordance with the present invention;

FIG. 3 is a top view of the ring holder with parts broken away; and

FIG. 4 is an end view of the ring holder with parts broken away.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

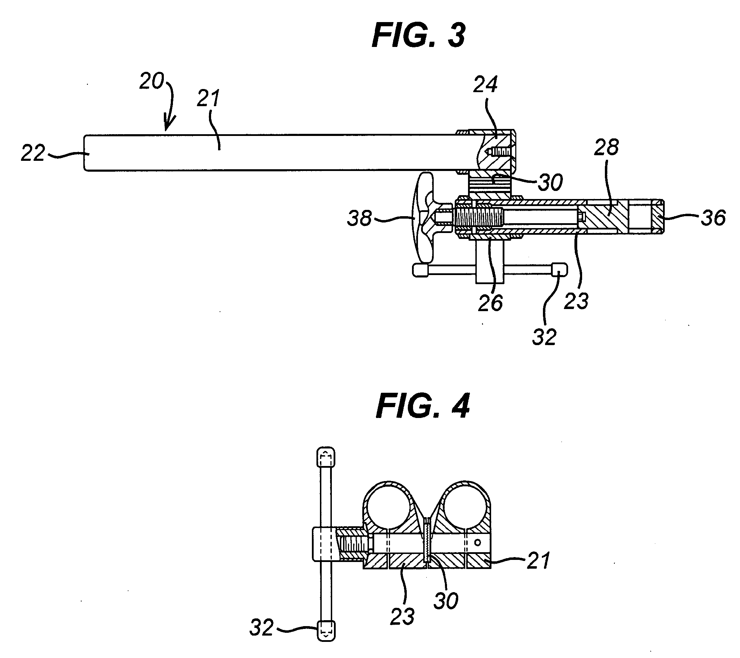

The present invention provides methods and devices for holding a retractor ring. A retractor ring holder 20 is illustrated in Figs. 2-4. Holder 20 includes a bar portion 21 and a ring holder portion 23. Bar portion 21 has a proximal end 22 and a distal end 24. Similarly, ring holder portion 23 includes a proximal end 26 and a distal end 28. Proximal end 22 connects to a table post 12 or to a horizontal extension bar (not shown) in a manner known to those skilled in the art. Referring now to Fig. 4, distal end 24 of bar portion 21 is selectively fixedly connected to ring holder portion 23 by an annual mating serrated saw-like connection 30, which may also be referred to as a starburst connection. Connection 30 is selectively loosened and tightened by rotating handle 32 in either a clockwise or counter-clockwise direction. In the loosened position, ring holder portion 23 is free to rotate 360 degrees with respect to bar portion 21. In the tightened position, however, ring holder portion 23 is fixedly connected to bar portion 21.

. Ring holder portion 23 includes a fixed clamp 34 and an axially moveable clamp 36, each disposed at distal end 28 of ring holder portion 23. Clamps 34, 36 are designed to selectively connect distal end 28 of ring holder portion to a ring 10 via rotation of handle 38 in either a clockwise or counter-clockwise direction.

The combination of connection 30 and the connection to the ring made by clamps 34, 36 provide an extremely strong connection, one that can withstand a pulling force on the ring up to approximately 250 pounds, without movement occurring between ring 10 and holder 20. Thus, the chance of an unintended disengagement of the ring from the ring holder is reduced.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A retractor ring holder comprising:
a bar portion having a proximal end and a distal end;
a ring holder portion having a proximal end and a distal end, said distal end having a fixed clamp portion and a moveable clamp portion;
an annual mating saw connection being disposed between said distal end of said bar portion and said proximal end of said ring holder portion; and
a first handle being attached to said connection to selectively place said bar portion and said ring holder portion in one of a fixed position with respect to each other and a moveable position, in said moveable position said ring holder portion is free to rotate 360° with respect to said bar portion.

2. The retractor ring holder according to claim 1, further comprising a second handle being attached to said ring holder portion proximal end to selectively move said moveable clamp portion with respect to said fixed clamp portion.

3. The retractor ring holder according to claim 1, wherein said first handle is rotatable.

4. The retractor ring holder to claim 1, wherein said bar portion is linear.

5. The retractor ring holder according to claim 1, wherein said second handle is rotatable.
